# EUROPEAN PATENT SPECIFICATION

(11) **EP 3 723 759 B1**
(45) Date of publication and mention of the grant of the patent: **10.01.2024**
(21) Application number: 18887667.6
(22) Date of filing: 14.12.2018
(51) Int. Cl.: A61K 31/517, A61P 25/24

(54) **TREATMENT OF POST-TRAUMATIC SYNDROME DISORDER**
BEHANDLUNG VON ZUSTÄNDEN DES POSTTRAUMATISCHEN SYNDROMS
TRAITEMENT D'UN TROUBLE DE SYNDROME POST-TRAUMATIQUE

(30) Priority: 14.12.2017 US 201762598757 P
(43) Date of publication of application: 21.10.2020
(73) Proprietor: Aché Laboratórios Farmacêuticos S.A., 07034-904 Guarulhos (BR)
(72) Inventor: MASCARELLO, Alessandra, 05049- 000 São Paulo - SP (BR); GUIMARÃES, Cristiano Ruch Werneck, 04087-002 São Paulo - SP (BR); AZEVEDO, Hatylas Felype Zaneti de, 01545-010 São Paulo - SP (BR); JUNIOR, Marcos Antonio Ferreira, 04127-000 São Paulo SP (BR)
(74) Representative: KATZAROV S.A.
(86) International application number: PCT/BR2018/050461
(87) International publication number: WO 2019/113668

(56) References cited:
- EP-A1- 3 130 339
- WO-A1-2012/037634
- US-B2- 9 181 201
- KOOLA, MM et al.: "High-dose prazosin for the treatment of post-traumatic stress disorder", Therapeutic Advances in Psychopharmacology, vol. 4, no. 1 16 August 2013 (2013-08-16), pages 43-47, XP055672838,

## Description

The present invention broadly concerns the treatment of Post-Traumatic Syndrome Disorder, known as PTSD.

### PRIOR ART

Treating mental health illnesses has always been a challenge, because of all the complexity involved, both from the psychiatric/psychological side and the medication side, and the difficult balance between them.

While the psychiatric/psychological treatment requires highly skilled professionals to detect the symptoms of each disease, as well as the co-existence of diseases (such as anxiety disorders, mood disorders, psychotic disorders, eating disorders, impulse control and addiction disorders, personality disorders, obsessive-compulsive disorder, post-traumatic stress disorder, stress response syndromes, dissociative disorders, factitious disorders, sexual and gender disorders, tic disorders, etc.), psychiatric medication does not cure mental illnesses, even if it can significantly improve symptoms.

Presently, regardless of what they are and how they manifest themselves, most of those disorders are treated with antidepressant and/or anti-stress medication.

This invention is specifically related to medication for post-traumatic stress disorder (PTSD), which is a chronic anxiety disorder that follows an exposure to traumatic events. Nearly everyone will experience a range of reactions after trauma, yet most people recover from initial symptoms naturally. Those who continue to experience problems may be diagnosed with PTSD. People who have PTSD may feel stressed or frightened even when they are not in danger.

Not every traumatized person develops ongoing (chronic) or even short-term (acute) PTSD. Not everyone with PTSD has been through a dangerous event. Some experiences, like the sudden, unexpected death of a loved one, can also cause PTSD. Symptoms usually begin early, within 3 months of the traumatic incident, but sometimes they begin years afterward. To be considered a case of PTSD, symptoms must last for more than one month and be severe enough to interfere with relationships or work. The course of the illness varies. Some people recover within 6 months, while others have symptoms that last much longer. In some people, the condition becomes chronic. Very typically, war veterans are affected by PTSD.

The most commonly used medications for PTSD are well known antidepressants, which relieve symptoms of depression and anxiety. Selective serotonin reuptake inhibitors (for example, fluoxetine) are typically the first line treatment, and are often prescribed for the treatment of PTSD. Tricyclic antidepressants (for example, de-sipramine) or monoamine oxidase inhibitors are generally resorted as second- and third-line strategies due to tolerability issues.

Other anti-depression drugs have also been recommended to treat PTSD, such as benzodiazepines. No data is known to support the efficacy of benzodiazepines for the treatment of what is considered "core" PTSD symptoms such as avoidance, hyperarousal, numbing and dissociation. Nevertheless, they are commonly prescribed, probably because they presumably manage secondary symptoms of PTSD such as insomnia and anxiety due to their rapid short-term symptomatic relief.

Unfortunately, many PTSD patients fail to adequately respond to the existing pharmacological treatments with only about 60% patients responding to treatment to some degree and approximately 20-30% who achieve full remission. Thus, it seems that the available pharmacotherapies do not offer a sufficient solution for PTSD patients and there is a major need for novel treatment strategies.

Additionally, because antidepressant medication constitutes the first line pharmacological treatment for PTSD and many patients display no beneficial drug effects, it has been suggested that combinations of antidepressants with additional drugs may be necessary. Indeed, the heterogeneity of symptom clusters in PTSD, as well as the complex psychiatric comorbidities (for example, with depression or substance abuse), further support the notion that combinations of medications are in need. Therefore, the present consensus for effective treatment of PTSD and its complex psychiatric comorbidities is a combination of treatments.

However, a combination of medications to treat complex behavior disorders such as PTSD is not without drawbacks, as one has to deal with two sets of drug side effects instead of one as well as drug-drug interactions.

Thus, it seems that the available pharmacotherapies do not offer a sufficient solution for PTSD patients and there is a major need for novel treatment strategies.

### DETAILS OF THE INVENTION

A research investigation found that a certain quinazolinone compound provides effective treatment for PTSD symptoms, more effectively than drugs presently employed in such a treatment, with fewer side effects.

The quinazolinone compound of the invention is 3-(2-(4-(2-methoxyphenyl)piperazine-1-yl) ethyl) quinazoline-4(3H)-one, with the structure below:

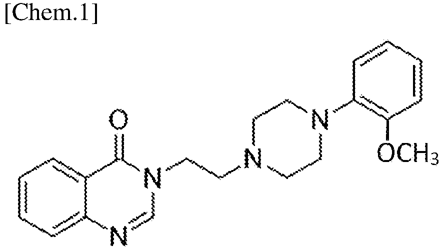

Within the meaning of the quinazolinone compound of the invention are also included pharmaceutically acceptable salts, enantiomers, diasteroisomers, stereoisomers, crystals, hydrates and solvates thereof, provided they significantly afford the activity of the compound concerning PTSD treatment. Within the disclosure of the quinazolinone compound of the invention are also included prodrugs and metabolites thereof, as well as the free base molecule with minor modifications (e.g. inclusion of substituents), which, as known to a person having skill in the art, do not significantly modify the pharmacophore of the molecule towards PTSD.

Without excluding any other alternatives, adequate salts of the quinazolinone compound of the invention are pharmaceutically acceptable acid addition salts thereof, such as inorganic addition salts e.g. hydrochloride, hydrobromide, sulfate, nitrate, phosphate, and pharmaceutically acceptable organic additions salts such as acetate, propionate, hexanoate, heptanoate, glycolate, piruvate, lactate, malonate, malate, maleate, fumarate, tartrate, citrate, succinate, mesylate, acistrate, besylate, tosylate, xinafoate, benzoate, p-toluenesulfonate, cinnamate, p-chlorobenzenesulfonate, 2-naphtalenesulfonate, p-toluenesulphonate, camphorsulfonate, trimethylacetate, t-butylacetate, laurylsulphate, gluconate, glutarate, hydroxynaphthoate, salicylate, stearate, muconate, mandelate, 2-hydroxyethanesulfonate.

Without excluding any other alternatives, adequate salts of the quinazolinone compound of the invention are pharmaceutically acceptable alkaline addition salts thereof, for instance alkaline metal salts such as sodium, potassium, lithium, calcium, magnesium, bismuth, bromide, or pharmaceutically acceptable salts with organic bases such as primary, secondary or tertiary amines (e.g. isopropylamine, diethylamine, trimethylamine, triisopropylamine, tri-n-propylamine, ethanolamine, 2-dimethylaminoethanol, tromethamine, N-methyl-D-glucamine) and with aminoacid salts such as arginine, lysine, histidine, caffeine, procaine, hydro amine, choline, betaine, ethylene diamine, glucosamine, theobromine, purine, morphine.

The quinazolinone compound or the invention can be prepared by known methods. For instance it can be prepared as illustrated in diagram 1 below:

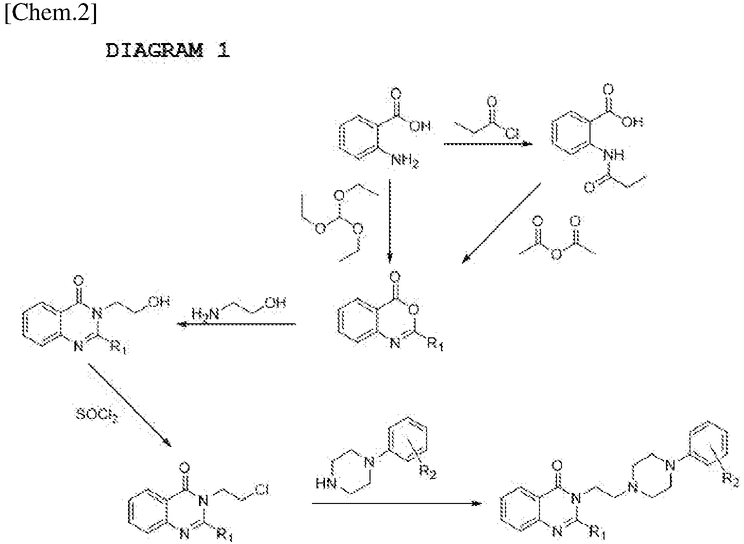

Wherein R1 is hydrogen and R2 is methoxyphenyl.

Pursuant to diagram 1, the compound of the invention may be obtained from anthranilic acid, using ethyl orthoformate and monoethanolamine for the formation of the intermediate 3- (2-hydroxyethyl)quinazoline-4 (3H)-one. The reaction of this intermediate with thionyl chloride results in the formation of a chlorinated derivative, which is subsequently condensed with a substituted piperazine phenyl resulting in the desired compound. Other synthetic alternatives may be used to obtain it, without adversely affecting the outcome.

One aspect of the invention is the quinazolinone compound as defined above for use in the treatment of PTSD characterized by the administration of a therapeutically efficient amount of the quinazolinone compound to a person in need thereof.

Another aspect of the invention is a pharmaceutical composition for use in the treatment of PTSD comprising the quinazolinone compound as defined above, and one or more excipients.

The selection of excipients to be used or preparing pharmaceutical compositions is generally undertaken by considering the type of administration pathway, the physical and chemical compatibility of the excipient with the active ingredient, the manner of preparing the pharmaceutical presentation and the effects on its efficacy. These excipients are known in the art and are described in the literature (for instance in: Handbook of Pharmaceutical Manufacturing Formulations - Vol. 1 a 6 - 2004 - Sarfaraz K. Niazi - CRC Press and Remington's Pharmaceutical Sciences, Mack Publishing), widely used by technical professionals in the matter.

For therapeutic use and administration, the quinazolinone compound of the invention may be formulated in compositions appropriate for oral, parenteral, nasal, rectal, transmucosal and transdermal administration, using conventional techniques and appropriate excipients.

There are no specific restrictions as to the dosage forms comprising the quinazolinone compound of the invention. For instance, as a solid for oral administration adequate dosage forms may be tablets, pills, dragées, capsules, granules, powders, pellets, lyophilizates and similar presentations. As a liquid for oral administration, adequate dosage forms may be solutions, dispersions, suspensions, emulsions, oils, syrups, etc. Liquid dosage forms may be used for injections, such as intravenous, intramuscular, subcutaneous and intradermal.

Other examples of dosage forms comprise liposomes and nanoparticles, or any other known administration aspects to a person skilled in the art. The dosage form may provide immediate, controlled or delayed release of the quinazolinone compound of the invention.

Another aspect of the invention are dosage forms, for use in the treatment of PTSD, as described above, containing 0.01 a 5000 mg of the quinazolinone compound of the invention, for example, and may be administered once or more times a day, during the treatment.

In a particular embodiment, the composition of the invention may comprise, other than the quinazolinone compound, at least another active principle, for instance chosen from anti-depressant, anxiolytic, anti-retroviral, anti-cancer, anti-AIDS, antibiotic, antidiabetic, anti-hypertensive, analgesic, anti-inflammatory.

In another aspect, the invention concerns the quinazolinone compound described above for use in the treatment of PTSD.

### Brief Description of Drawings

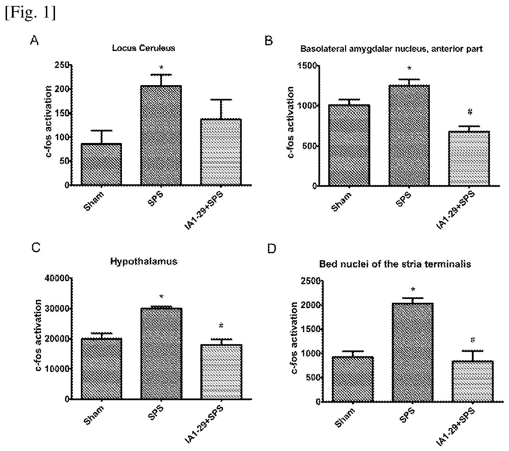

[fig.1]
- Count of c-fos positive neurons in brain regions. The brain regions exemplified in Figure 1 are locus ceruleus (Figure 1A), amygdala (Figure 1B), hypothalamus (Figure 1C) and bed nuclei stria terminalis (Figure 1D). The experiment comprised three different groups (n = 10 per group): mice not subjected to the Single-Prolonged Stress protocol (Sham), mice subjected to the Single-Prolonged Stress protocol (SPS) and mice subjected to the Single-Prolonged Stress protocol but pre-treated with 3-(2-(4-(2-methoxyphenyl)piperazine-1-yl) ethyl) quinazoline-4(3H)-one, named IA1-29, at 30 mg/kg (IA1-29 + SPS).

### EXAMPLE

The quinazolinone of the invention was named IA1-29 and was tested in an *in vivo* model that mimics the activation that occurs in the brain after a prolonged stress stimulus..

The effects of the administration of IA1-29 at 30 mg/kg p.o. in brain activation were assessed in mice subjected to the Single Prolonged Stress (SPS) model. The SPS protocol is a well established model of PTSD that combines multiple stressors (physical restraint, forced swim, predator scent and ether anesthesia (Borghans & Homberg, 2015; Yamamoto et al., 2009)). The activation of specific brain regions was determined by counting the number of neurons expressing the c-fos protein, an immediate early gene product that can be used as a marker of cell activation in individual neurons. Interestingly, the SPS-Induced c-fos expression in key brain regions involved in stress response was supressed by the prior administration of IA1-29. The count of c-fos positive neurons in some of these regions can be visualized in Figure 1. The experiment comprised three different groups (n = 10 per group): mice not subjected to the SPS protocol (Sham), mice subjected to the SPS model (SPS) and animals submitted to the SPS protocol but pre-treated with IA1-29 at 30 mg/kg (IA1-29 + SPS). The brain regions exemplified in Figure 1 are locus ceruleus (Figure 1A), amygdala (Figure 1B), hypothalamus (Figure 1C) and bed nuclei stria terminalis (Figure 1D). The statistical analysis between the groups was performed by ANOVA followed by bonferroni post-hoc test. * p < 0.05 between SPS and Sham groups. # p < 0.05 between IA1-29+SPS and SPS groups.

A person having skills in the concerned art, by way of the explanations and examples comprised herein will promptly appreciate the advantages of the invention, and will be able to propose equivalent embodiments of the invention without departing from the scope of the attached inventions.

## Claims

1. 3-(2-(4-(2-methoxyphenyl)piperazine-1-yl) ethyl) quinazoline-4(3H)-one for use in the treatment of Post-Traumatic Syndrome Disorder (PTSD).

2. 3-(2-(4-(2-methoxyphenyl)piperazine-1-yl) ethyl) quinazoline-4(3H)-one for use according to claim 1, wherein 3-(2-(4-(2-methoxyphenyl)piperazine-1-yl) ethyl) quinazoline-4(3H)-one is as pharmaceutically acceptable salts.

3. 3-(2-(4-(2-methoxyphenyl)piperazine-1-yl) ethyl) quinazoline-4(3H)-one for use according to claim 1, wherein 3-(2-(4-(2-methoxyphenyl)piperazine-1-yl) ethyl) quinazoline-4(3H)-one has modifications that do not significantly modify the pharmacophore thereof towards the treatment of Post-Traumatic Syndrome Disorder.

4. 3-(2-(4-(2-methoxyphenyl)piperazine-1-yl) ethyl) quinazoline-4(3H)-one for use according to claim 2, wherein the pharmaceutically acceptable salts are chosen from the inorganic addition salts hydrochloride, hydrobromide, sulfate, nitrate, phosphate; from the organic additions salts acetate, propionate, hexanoate, heptanoate, glycolate, piruvate, lactate, malonate, malate, maleate, fumarate, tartrate, citrate, succinate, mesylate, acistrate, besylate, tosylate, xinafoate, benzoate, p-toluenesulfonate, cinnamate, p-chlorobenzenesulfonate, 2-naphtalenesulfonate, p-toluenesulphonate, camphorsulfonate, trimethylacetate, t-butylacetate, laurylsulphate, gluconate, glutarate, hydroxynaphthoate, salicylate, stearate, muconate, mandelate, 2-hydroxyethanesulfonate; and from the alkaline addition salts of metals sodium, potassium, lithium, calcium, magnesium, bismuth, bromide; and from or pharmaceutically acceptable salts with the organic bases primary, secondary or tertiary amines; and salts with the amino acid arginine, lysine, histidine, caffeine, procaine, hydrobamine, choline, betaine, ethylenediamine, glycosamine, teobromine, purine, morpholine.

5. 3-(2-(4-(2-methoxyphenyl)piperazine-1-yl) ethyl) quinazoline-4(3H)-one for use according to any one of claims 1-4, wherein treatment of Post-Traumatic Syndrome Disorder comprises the administration of a therapeutically efficient amount of the quinazolinone compound as defined in any one of claims 1-4 to a person in need thereof.

6. A pharmaceutical composition for use in a method for the treatment according to claim 1, wherein the pharmaceutical composition comprises the quinazolinone compound as defined in any one of claims 1 to 4, and one or more excipients.

7. The pharmaceutical composition for use according to claim 6 comprising, other than the quinazolinone compound as defined in any one of claims 1 to 4, at least another active principle, chosen from anti-depressant, anxiolytic, anti-retroviral, anti-cancer, antibiotic, antidiabetic, anti-hypertensive, analgesic, anti-inflammatory.

8. Dosage forms for use in a method for the treatment according to claim 1, wherein the dosage forms comprise 0.01 a 5000 mg of the quinazolinone compound as defined in any one of claims 1 to 4.

## Patentansprüche

1. 3-(2-(4-(2-Methoxyphenyl)piperazin-1-yl)ethyl)chinazolin-4(3H)-on zur Verwendung bei der Behandlung eines posttraumatischen Störungssyndroms (PTSS) .

2. 3-(2-(4-(2-Methoxyphenyl)piperazin-1-yl)ethyl)chinazolin-4(3H)-on zur Verwendung nach Anspruch 1, wobei das 3-(2-(4-(2-Methoxyphenyl)piperazin-1-yl)ethyl)chinazolin-4(3H)-on in Form von pharmazeutisch verträglichen Salzen ist.

3. 3-(2-(4-(2-Methoxyphenyl)piperazin-1-yl)ethyl)chinazolin-4(3H)-on zur Verwendung nach Anspruch 1, wobei das 3-(2-(4-(2-Methoxyphenyl)piperazin-1-yl)ethyl)chinazolin-4(3H)-on Modifikationen aufweist, die den Pharmakophor für die Behandlung des posttraumatischen Störungssyndroms nicht wesentlich modifizieren.

4. 3-(2-(4-(2-Methoxyphenyl)piperazin-1-yl)ethyl)chinazolin-4(3H)-on zur Verwendung nach Anspruch 2, wobei die pharmazeutisch verträglichen Salze aus den anorganischen Additionssalzen Hydrochlorid, Hydrobromid, Sulfat, Nitrat, Phosphat; aus den organischen Additionssalzen Acetat, Propionat, Hexanoat, Heptanoat, Glycolat, Piruvat, Lactat, Malonat, Malat, Maleat, Fumarat, Tartrat, Citrat, Succinat, Mesylat, Acistrat, Besylat, Tosylat, Xinafoat, Benzoat, p-Toluolsulfonat, Cinnamat, p-Chlorbenzolsulfonat, 2- Naphtalensulfonat, p-Toluolsulfonat, Camphorsulfonat, Trimethylacetat, t-Butylacetat, Laurylsulfat, Gluconat, Glutarat, Hydroxynaphthoat, Salicylat, Stearat, Muconat, Mandelat, 2-Hydroxyethansulfonat; und aus den alkalischen Additionssalzen der Metalle Natrium, Kalium, Lithium, Calcium, Magnesium, Wismut, Bromid; und aus pharmazeutisch verträglichen Salzen mit den organischen Basen primäre, sekundäre oder tertiäre Amine; und Salzen mit der Aminosäure Arginin, Lysin, Histidin, Koffein, Procain, Hydrobamin, Cholin, Betain, Ethylendiamin, Glucosamin, Teobromin, Purin, Morpholin ausgewählt sind.

5. 3-(2-(4-(2-Methoxyphenyl)piperazin-1-yl)ethyl)chinazolin-4(3H)-on zur Verwendung nach einem der Ansprüche 1 bis 4, wobei die Behandlung des posttraumatischen Störungssyndroms die Verabreichung einer therapeutisch wirksamen Menge der Chinazolinon-Verbindung nach einem der Ansprüche 1 bis 4 an eine Person mit Bedarf daran umfasst.

6. Pharmazeutische Zusammensetzung zur Verwendung in einem Verfahren nach Anspruch 1, wobei die pharmazeutische Zusammensetzung die Chinazolinon-Verbindung nach einem der Ansprüche 1 bis 4 und einen oder mehrere Exzipienten umfasst.

7. Pharmazeutische Zusammensetzung zur Verwendung nach Anspruch 6, umfassend neben der Chinazolinon-Verbindung nach einem der Ansprüche 1 bis 4 mindestens ein weiteres Wirkprinzip, das aus Antidepressiva, Anxiolytika, antiretroviralen Mitteln, krebshemmenden Mitteln, Antibiotika, Antidiabetika, blutdrucksenkenden Mitteln, Analgetika, entzündungshemmenden Mitteln gewählt ist.

8. Darreichungsformen zur Verwendung in einem Verfahren nach Anspruch 1, wobei die Darreichungsformen 0,01 bis 5000 mg der Chinazolinon-Verbindung nach einem der Ansprüche 1 bis 4 umfassen.

## Revendications

1. 3-(2-(4-(2-méthoxyphényl)pipérazine-1-yl)éthyl) quinazoline-4(3H)-one pour une utilisation dans le traitement du trouble du syndrome post-traumatique **(PTSD).**

2. 3-(2-(4-(2-méthoxyphényl)pipérazine-1-yl)éthyl) quinazoline-4(3H)-one pour une utilisation selon la revendication 1, dans laquelle la 3-(2-(4-(2-méthoxyphényl)pipérazine-1-yl)éthyl)quinazoline-4(3H)-one se présente sous forme de sels pharmaceutiquement acceptables.

3. 3-(2-(4-(2-méthoxyphényl)pipérazine-1-yl)éthyl) quinazoline-4(3H)-one pour une utilisation selon la revendication 1, dans laquelle la 3-(2-(4-(2-méthoxyphényl)pipérazine-1-yl)éthyl)quinazoline-4(3H)-one a des modifications qui ne modifient pas de manière significative son pharmacophore pour le traitement du trouble du syndrome post-traumatique.

4. 3-(2-(4-(2-méthoxyphényl)pipérazine-1-yl)éthyl) quinazoline-4(3H)-one pour une utilisation selon la revendication 2, dans laquelle les sels pharmaceutiquement acceptables sont choisis parmi les sels d'addition inorganiques chlorhydrate, bromhydrate, sulfate, nitrate, phosphate ; parmi les sels d'addition organiques acétate, propionate, hexanoate, heptanoate, glycolate, piruvate, lactate, malonate, malate, maléate, fumarate, tartrate, citrate, succinate, mésylate, acistrate, besylate, tosylate, xinafoate, benzoate, p-toluènesulfonate, cinnamate, p-chlorobenzènesulfonate, 2-naphtalènesulfonate, p-toluènesulfonate, camphorsulfonate, triméthylacétate, t-butylacétate, laurylsulfate, gluconate, glutarate, hydroxynaphtoate, salicylate, stéarate, muconate, mandélate, 2-hydroxyéthanesulfonate ; et parmi les sels d'addition alcalins de métaux sodium, potassium, lithium, calcium, magnésium, bismuth, bromure ; et parmi les sels pharmaceutiquement acceptables avec les bases organiques amines primaires, secondaires ou tertiaires ; et les sels avec l'acide aminé arginine, lysine, histidine, caféine, procaïne, hydrobamine, choline, bétaïne, éthylènediamine, glycosamine, théobromine, purine et morpholine.

5. 3-(2-(4-(2-méthoxyphényl)pipérazine-1-yl)éthyl) quinazoline-4(3H)-one pour une utilisation selon l'une quelconque des revendications 1 à 4, dans laquelle le traitement du trouble du syndrome post-traumatique comprend l'administration d'une quantité thérapeutiquement efficace du composé de quinazolinone tel que défini dans l'une des revendications 1 à 4 à une personne en ayant besoin.

6. Composition pharmaceutique pour une utilisation dans un procédé de traitement selon la revendication 1, dans laquelle la composition pharmaceutique comprend le composé de quinazolinone tel que défini dans l'une quelconque des revendications 1 à 4, et un ou plusieurs excipients.

7. Composition pharmaceutique pour une utilisation selon la revendication 6 comprenant, outre le composé de quinazolinone tel que défini dans l'une quelconque des revendications 1 à 4, au moins un autre principe actif, choisi parmi un antidépresseur, un anxiolytique, un antirétroviral, un agent anticancéreux, un antibiotique, un antidiabétique, un antihypertenseur, un analgésique et un anti-inflammatoire.

8. Formes posologiques pour une utilisation dans un procédé de traitement selon la revendication 1, dans lesquelles les formes posologiques comprennent 0,01 à 5000 mg du composé de quinazolinone tel que défini dans l'une quelconque des revendications 1 à 4.
